(19) Europäisches Patentamt
European Patent Office
Office européen des brevets

(11) **EP 4 036 536 A1**

(12) **EUROPEAN PATENT APPLICATION**
published in accordance with Art. 153(4) EPC

(43) Date of publication:
**03.08.2022 Bulletin 2022/31**

(21) Application number: **20867557.9**

(22) Date of filing: **23.06.2020**

(51) International Patent Classification (IPC):
**G01G 17/00** (2006.01)     **G01G 9/00** (2006.01)
**G01G 23/35** (2006.01)

(52) Cooperative Patent Classification (CPC):
**G01G 9/00; G01G 17/00; G01G 23/35**

(86) International application number:
**PCT/JP2020/024583**

(87) International publication number:
**WO 2021/059631 (01.04.2021 Gazette 2021/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2019 JP 2019172491**

(71) Applicant: **NEC Corporation**
**108-8001 Tokyo (JP)**

(72) Inventor: **NASU, Yasuyuki**
**Tokyo 108-8001 (JP)**

(74) Representative: **Betten & Resch**
**Patent- und Rechtsanwälte PartGmbB**
**Maximiliansplatz 14**
**80333 München (DE)**

(54) **INDIVIDUAL BODY WEIGHT ESTIMATION SYSTEM, METHOD, AND PROGRAM**

(57) An imaging unit 81 images a plurality of individuals whose body weight is to be measured in a batch as a subject. An individual body length measurement unit 82 measures a first length indicating a size of each individual included in the plurality of individuals from a captured image. A body weight rate calculation unit 83 calculates a body weight rate of each individual from the measured first length of each individual. A body weight estimation unit 84 estimates a body weight of each individual, based on the body weight of the plurality of individuals and the body weight rate of each individual.

FIG. 5

**Description**

**Technical Field**

The present invention relates to an individual body weight estimation system, an individual body weight estimation method, and an individual body weight estimation program for estimating a body weight of an individual.

**Background Art**

When measuring body weights of individuals, it is common to use a scale to directly measure the body weight of each individual. However, there are various situations in which it is not possible to directly measure each individual using a scale. For example, when observing growth of individual fishes being cultured, it is difficult to weigh each fish on a scale. A method to solve such a problem is described in patent literature 1, for example.

Specifically, patent literature 1 describes a method for measuring the length of an object from a photographic image of the object to be measured. In the method described in patent literature 1, the coordinates in coordinate space of the head and tail of the fish to be measured are identified using a triangulation method, and a length between characteristic parts forming a pair is calculated as a length of the fish, using the spatial coordinates of the identified characteristic parts of the fish to be measured. The patent literature 1 also describes estimating the body weight of a fish based on its length.

**Citation List**

**Patent Literature**

PTL 1: International Patent Publication No. 2019/045089

**Summary of Invention**

**Technical Problem**

In addition to the situations described above, there are also situations in which the body weight of a plurality of individuals is measured together, making it difficult to directly measure the body weight of each individual. For example, in the trading of farmed tuna, a plurality of pieces of tuna are weighed together on a ship using a crane scale. At that time, the body weight of each individual tuna is measured by the eyes of persons related to the transaction, making it difficult for buyers who are not familiar with negotiations to make a proper trading.

Since the method described in patent literature 1 does not assume a situation where body weight is measured in such a batch, it is difficult to say that the body weight of an individual fish can be estimated with high accuracy simply by calculating the length of the individual fish.

Therefore, it is an object of the present invention to provide an individual body weight estimation system, an individual body weight estimation method, and an individual body weight estimation program that can estimate a body weight of each individual with high accuracy, even when a body weight of a plurality of individuals is measured in a batch.

**Solution to Problem**

An individual body weight estimation system according to the present invention includes an imaging unit which images a plurality of individuals whose body weight is to be measured in a batch as a subject, an individual body length measurement unit which measures a first length indicating a size of each individual included in the plurality of individuals from a captured image, a body weight rate calculation unit which calculates a body weight rate of each individual from the measured first length of each individual, and a body weight estimation unit which estimates a body weight of each individual, based on the body weight of the plurality of individuals and the body weight rate of each individual.

An individual body weight estimation method according to the present invention includes imaging a plurality of individuals whose body weight is to be measured in a batch as a subject, measuring a first length indicating a size of each individual included in the plurality of individuals from an captured image, calculating a body weight rate of each individual from the measured first length of each individual, and estimating a body weight of each individual, based on the body weight of the plurality of individuals and the body weight rate of each individual.

An individual body weight estimation program according to the present invention causes a computer to execute an individual length measurement process of measuring a first length indicating a size of each individual included in a plurality of individuals from an image, captured by an imaging unit, with the plurality of individuals whose body weight is to be measured in a batch as a subject, a body weight rate calculation process of calculating a body weight rate of each individual from the measured first length of each individual, and a body weight estimation process of estimating a body weight of each individual based on the body weight of the plurality of individuals and the body weight rate of each individual.

**Advantageous Effects of Invention**

According to the present invention, it is possible to estimate a body weight of each individual with high accuracy, even when a body weight of a plurality of individuals is measured in a batch.

**Brief Description of Drawings**

[FIG. 1] It depicts a block diagram showing a configuration example of the first example embodiment of an individual body weight estimation system according to the present invention.
[FIG. 2] It depicts a flowchart showing an operation example of an individual body weight estimation system of the first example embodiment.
[FIG. 3] It depicts a block diagram showing a configuration example of the second example embodiment of an individual body weight estimation system according to the present invention.
[FIG. 4] It depicts a flowchart showing an operation example of an individual body weight estimation system of the second example embodiment.
[FIG. 5] It depicts a block diagram showing an overview of an individual body weight estimation system according to the present invention.

**Description of Embodiments**

Hereinafter, example embodiments of the present invention are described with reference to the drawings. In those example embodiments, tuna will be used as an example to illustrate the specific individual to be measured. This is because, due to the commercial practice of tuna trading, it is common for a plurality of fishes to be weighed together. However, an individual is not limited to a tuna, and the target is arbitrary if there is a possibility that a plurality of individuals will be measured together.

Example embodiment 1.

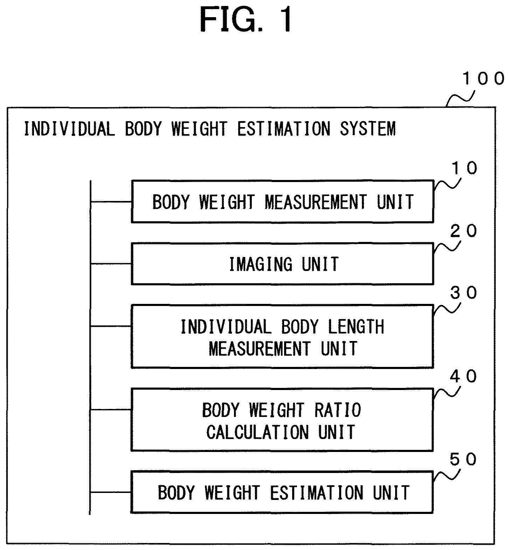

FIG. 1 is a block diagram showing a configuration example of the first example embodiment of an individual body weight estimation system according to the present invention. The individual body weight estimation system 100 of this example embodiment comprises a body weight measurement unit 10, an imaging unit 20, an individual body length measurement unit 30, a body weight rate calculation unit 40, and a body weight estimation unit 50.

The body weight measurement unit 10 measures a body weight of a plurality of individuals in a batch. Specifically, the body weight measurement unit 10 measures a body weight of a plurality of individuals in a batch by hanging. The body weight measurement unit 10 is realized, for example, by suspension scale such as a hoist scale, a crane scale, or the loke.

The imaging unit 20 is a device that images a plurality of individuals as a subject. In this example embodiment, the imaging unit 20 is realized by a device capable of imaging information that enables the individual body length measurement unit 30 described below to measure various lengths indicating size of the individuals. When the imaging unit 20 is realized by a stereo camera, the information in a depth direction of a plurality of individuals can also be recorded.

The mode of the imaging unit 20 is not limited to a stereo camera, but can be any device that can measure various lengths that indicate the size of an individual. The imaging unit 20 may, for example, be realized by a combination of multiple devices (for example, a combination of a visible light camera and an infrared camera, a combination of a visible light camera and a laser device, etc.).

In the example shown in FIG. 1, the case where the individual body weight estimation system 100 comprises one imaging unit 20 is illustrated, but the number of imaging unit 20 is not limited to one, and may be two or more. By using a plurality of imaging unit 20 to image a plurality of individuals from a plurality of different directions, the accuracy of measuring the individuals can be improved.

Specifically, the imaging unit 20 images the entire image of a plurality of individuals. For example, when a plurality of imaging unit 20 can image the entire image, each imaging unit 20 may integrate the imaged images into the entire image. When the imaging unit 20 is capable of imaging only a partial range of the plurality of individuals, the imaging unit 20 may be moved by a user or the like to cover the periphery of the plurality of individuals, so that the entire image of the plurality of individuals may be imaged. By rotating the plurality of individuals while the imaging unit 20 is fixed, the imaging unit 20 may image the entire image of the plurality of individuals.

The individual body length measurement unit 30 measures the size of each individual included in the plurality of individuals from an imaged image. Specifically, the individual body length measurement unit 30 measures at least one length indicating the size for each individual included in the plurality of imaged individuals. More specifically, the individual body length measurement unit 30 measures the most characteristic length indicating the size of the individual as a first length.

For example, in the case of a fish such as a tuna, a fork length, a total length, and a body length are the most characteristic lengths that indicate the size of the individual fish. Therefore, the individual body length measurement unit 30 may measure the fork length, the total length, or the body length of each fish included in the plurality of imaged fishes as the first length.

In addition to the first length, the individual body length measurement unit 30 may preferably measure a second length that indicates size of the individual. For example, in the case of a fish such as a tuna, in addition to the above first length, a body depth can be said to be a length indicating the size of the individual. Therefore, the individual body length measurement unit 30 may measure the body depth of each fish included in the plurality of imaged fishes as the second length.

More preferably, the individual body length measurement unit 30 may measure a third length indicating size of the individual, in addition to the first length and the second length. For example, in the case of a fish such as a tuna, in addition to the above first and second lengths, a body width can be said to be a length indicating the size of the individual. Therefore, the individual body length measurement unit 30 may measure the body width of each fish included in the plurality of imaged fishes as the third length.

In this way, the accuracy of estimating the body weight can be improved by having the individual body length measurement unit 30 measure multiple types of lengths from the image imaged for a single individual. The individual body length measurement unit 30 can use general image recognition techniques, for example, to measure the length of each individual from the captured image to indicate the size of the individual.

The body weight rate calculation unit 40 calculates the weight rate of each individual from the measured first length of each individual. The body weight rate calculation unit 40 may calculate the rate of the first length of each individual as it is, as the body weight rate of each individual.

The body weight rate calculation unit 40 may estimate the body weight of each individual from the measured first length of each individual, and calculate the body weight rate of each individual from the estimated body weight of each individual. Specifically, the body weight rate calculation unit 40 may estimate the body weight of each individual using a model for estimating the body weight from the measured first length of each individual. The manner of the model to be estimated is arbitrary, and may be realized by a simple regression model including the first length as an explanatory variable, for example. The model may also include not only the first length but also the second length and the third length as explanatory variables.

For example, the equation of the model to predict the weight W may be defined in the following form.

$$W = \alpha \times \text{fork length} + \beta \times \text{body depth} + \gamma \times \text{body width} + \delta$$

The method of defining the model to be used is also arbitrary. The model may be defined based on the experience of the user or others, or the model may be defined by a model generated by machine learning using the combination of each of the above lengths and the body weight of the individual with that length as learning data.

The body weight estimation unit 50 estimates the body weight of each individual based on the body weight of the plurality of individuals measured by the body weight measurement unit 10 and the body weight rate of each individual. Specifically, the body weight estimation unit 50 can estimate the body weight of each individual as the value obtained by distributing the body weight of the plurality of individuals, based on the body weight rate of each individual.

The individual body length measurement unit 30, the body weight rate calculation unit 40, and the body weight estimation unit 50 are realized by a processor (for example, a CPU (Central Processing Unit), a GPU (Graphics Processing Unit)) of a computer that operates according to a program (individual body weight estimation program). For example, the program is stored in a storage unit (not shown) of the individual body weight estimation system 100, and the processor may read the program and operate according to the program as the individual body length measurement unit 30, the body weight rate calculation unit 40, and the body weight estimation unit 50. In addition, the function of the individual body weight estimation system 100 may be provided in a SaaS (Software as a Service) manner.

The individual body length measurement unit 30, the body weight rate calculation unit 40, and the body weight estimation unit 50 may be realized by dedicated hardware, respectively. In addition, some or all of each component of each device may be realized by a general-purpose or dedicated circuit (circuitry), a processor, etc. or a combination of these. They may be configured by a single chip or by multiple chips connected through a bus. Some or all of components of each device may be realized by a combination of the above-mentioned circuitry, etc. and a program.

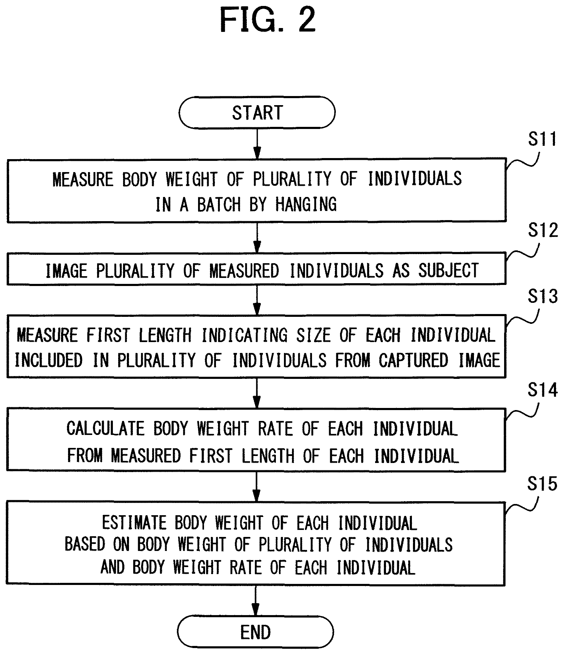

Next, the operation of the individual body weight estimation system 100 of this example embodiment will be described. FIG. 2 is a flowchart showing an operation example of the individual body weight estimation system 100 of this example embodiment. First, the body weight measurement unit 10 measures a body weight of a plurality of individuals in a batch by hanging (step S11). The imaging unit 20 images a plurality of measured individuals as a subject (step S12). The individual body length measurement unit 30 measures a first length indicating a size of each individual included in the plurality of individuals from a captured image, respectively (step S13). The body weight rate calculation unit 40 calculates a body weight rate of each individual from the measured first length of each individual (step S14). Then, the body weight estimation unit 50 estimates a body weight of each individual based on the body weight of the plurality of individuals and the body weight rate of each individual (step S15).

As described above, in this example embodiment, the imaging unit 20 images a plurality of individuals whose body weight is to be measured in a batch as a subject, and the individual body length measurement unit 30 measures a first length indicating a size of each individual included in the plurality of individuals from a captured image, respectively. Then, the body weight rate calculation unit 40 calculates a body weight rate of each individual from the measured first length of each individual, and the body weight estimation unit 50 estimates the body weight of each individual based on the body weight of the plurality of individuals and the body weight rate of each individual. Therefore, it is possible to estimate a body weight of each individual with high accuracy, even when a body weight of a plurality of individuals is measured in a batch.

Example embodiment 2.

Next, a second example embodiment of the individual body weight estimation system according to the invention will be described. In the first example embodiment, a case where the imaging unit 20 images a plurality of individuals as a subject is described. However, depending on the imaged angle, it may not be possible to image all the individuals to be measured. This example embodiment describes a method for detecting a situation where not all individuals are included in a captured image.

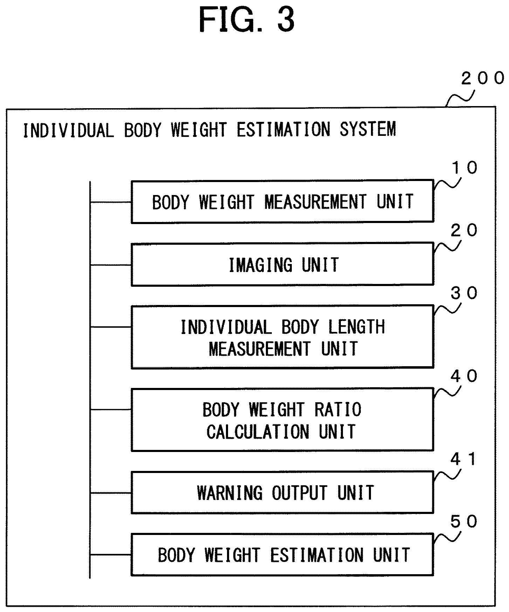

FIG. 3 is a block diagram showing a configuration example of the second example embodiment of an individual body weight estimation system according to the present invention. The same sign is attached to the same configuration as in the first example embodiment, and the explanation is omitted. The individual body weight estimation system 200 of this example embodiment comprises a body weight measurement unit 10, an imaging unit 20, an individual body length measurement unit 30, a body weight rate calculation unit 40, a warning output unit 41, and a body weight estimation unit 50.

In other words, the individual body weight estimation system 200 of this example embodiment differs from the individual body weight estimation system 100 of the first example embodiment in that the individual body weight estimation system 200 further comprises the warning output unit 41.

The warning output unit 41 calculates a sum of body weights of respective individuals estimated by the body weight rate calculation unit 40, and compares the sum with the body weight of a plurality of individuals measured by the body weight measurement unit 10. If a difference between the sum of the estimated body weights and the body weight of the plurality of measured individuals exceeds a predetermined threshold value, the warning output unit 41 outputs warning. The method by which the warning output unit 41 outputs warning is optional. The warning output unit 41 may display a warning message on a device such as a display (not shown), or output an audio or an alert.

The individual body length measurement unit 30, the body weight rate calculation unit 40, the warning output unit 41, and the body weight estimation unit 50 are realized by a processor of a computer that operates according to a program (individual body weight estimation program).

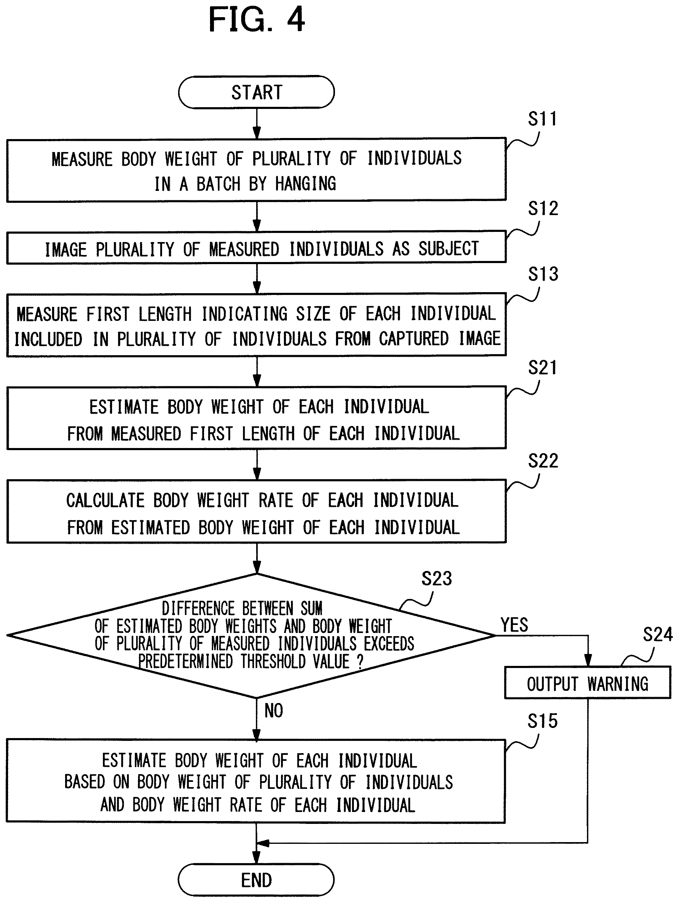

Next, the operation of the individual body weight estimation system 200 of this example embodiment will be described. FIG. 4 is a flowchart showing an operation example of the individual body weight estimation system 200 of this example embodiment. The processes up to the measurement of the first length of each individual are the same as the processes from step S11 to step S13 illustrated in FIG. 2.

The body weight rate calculation unit 40 estimates a body weight of each individual from the measured first length of each individual (step S21). Then, the body weight rate calculation unit 40 calculates a body weight rate of each individual from the estimated body weight of each individual (step S22).

The warning output unit 41 determines whether a difference between a sum of body weights of respective individuals estimated by the body weight rate calculation unit 40 and the body weight of the plurality of individuals measured by the body weight measurement unit 10 exceeds the predetermined threshold value (step S23). If the difference exceeds the threshold value (YES in step S23), the warning output unit 41 outputs warning (step S24). On the other hand, if the difference is less than the threshold value (NO in step S23), the process of step S15 illustrated in FIG. 2 is performed. Note that the process of step S22 is not performed before the process of step S23, but after the process of step S23.

As described above, in this example embodiment, the warning output unit 41 outputs warning when a difference between a sum of the estimated body weights of respective individuals and the measured body weight of the plurality of individuals exceeds a predetermined threshold value. Thus, in addition to the effect of the first example embodiment, it is possible to detect a presence of an individual that is not visible from the outside.

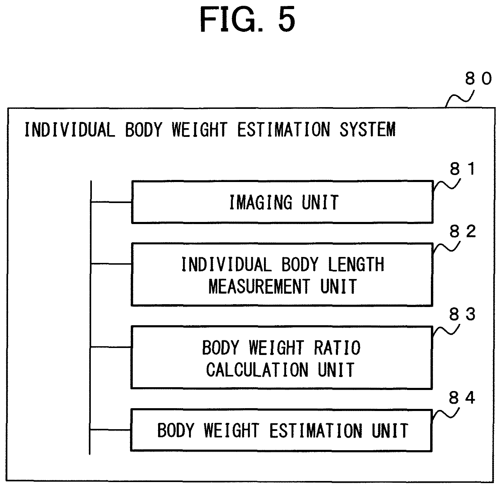

Next, an overview of the present invention will be described. FIG. 5 is a block diagram showing an overview of an individual body weight estimation system according to the present invention. An individual body weight estimation system 80 (for example, individual body weight estimation system 100, 200) according to the present invention comprises an imaging unit 81 (for example, the imaging unit 20) which images a plurality of individuals whose body weight is to be measured in a batch as a subject, an individual body length measurement unit 82 (for example, the individual body length measurement unit 30) which measures a first length indicating a size of each individual included in the plurality of individuals from a captured image, a body weight rate calculation unit 83 (for example, the body weight rate calculation unit 40) which calculates a body weight rate of each individual from the measured first length of each individual, and a body weight estimation unit 84 (for example, the body weight estimation unit 50) which estimates a body weight of each individual, based on the body weight of the plurality of individuals and the body weight rate of each individual.

With such a configuration, it is possible to estimate a body weight of each individual with high accuracy, even when a body weight of a plurality of individuals is measured in a batch.

The body weight rate calculation unit 83 may estimate the body weight of each individual from the measured first length of each individual, and calculate the weight rate of each individual from the estimated body weight of each individual.

The individual body weight estimation system 80 (for example, the individual body weight estimation system 200) may also comprise a warning output unit (for example, the warning output unit 41) which outputs warning when a difference between a sum of the estimated body weights of respective individuals and the measured body weight of the plurality of individuals exceeds a predetermined threshold value. With such a configuration, it is possible to detect a presence of an individual that is not visible from the outside.

The individual body length measurement unit 82 measures a second length indicating the size of each individual from the captured image, and the body weight rate calculation unit 83 estimates the body weight of each individual from the measured first length and the second length of each individual.

The imaging unit 81 may image the body weight of the plurality of individuals which is measured in a batch as the subject by hanging.

Specifically, the imaging unit 81 may image a plurality of tunas whose body weight is to be measured in a batch as a subject by a suspension scale, the individual body length measurement unit 82 may measure a fork length, a total length, or a body length of each tuna as the first length from the captured image, the body weight rate calculation unit 83 may calculate the body weight rate of each tuna from the measured first length of each tuna, and the body weight estimation unit 84 may estimate the body weight of each tuna based on the body weight of the plurality of tunas which is measured in a batch and the body weight rate of each tuna.

Although the present invention has been described with reference to the foregoing exemplary embodiments and examples, the present invention is not limited to the foregoing exemplary embodiments and examples. Various changes understandable by those skilled in the art can be made to the structures and details of the present invention within the scope of the present invention.

This application claims priority based on Japanese Patent Application No. 2019-172491 filed on September 24, 2019, the disclosure of which is incorporated herein in its entirety.

**Reference Signs List**

| | |
|---|---|
| 10 | Body weight measurement unit |
| 20 | Imaging unit |
| 30 | Individual body length measurement unit |
| 40 | Body weight rate calculation unit |
| 41 | Warning output unit |
| 50 | Body weight estimation unit |
| 100, 200 | Individual body weight estimation system |

**Claims**

1. An individual body weight estimation system comprising:

   an imaging unit which images a plurality of individuals whose body weight is to be measured in a batch as a subject,
   an individual body length measurement unit which measures a first length indicating a size of each individual included in the plurality of individuals from a captured image,
   a body weight rate calculation unit which calculates a body weight rate of each individual from the measured first length of each individual, and
   a body weight estimation unit which estimates a body weight of each individual, based on the body weight of the plurality of individuals and the body weight rate of each individual.

2. The individual body weight estimation system according to claim 1, wherein
   the body weight rate calculation unit estimates the body weight of each individual from the measured first length of each individual, and calculates the weight rate of each individual from the estimated body weight of each individual.

3. The individual body weight estimation system according to claim 2, further comprising
   a warning output unit which outputs warning when a difference between a sum of the estimated body weights of respective individuals and the measured body weight of the plurality of individuals exceeds a predetermined threshold value.

4. The individual body weight estimation system according to claim 2 or 3, wherein

   the individual body length measurement unit measures a second length indicating the size of each individual from the captured image, and
   the body weight rate calculation unit estimates the body weight of each individual from the measured first length and the second length of each individual.

5. The individual body weight estimation system according to any one of claims 1 to 4, wherein
   the imaging unit images the body weight of the plurality of individuals which is measured in a batch as the subject by hanging.

6. The individual body weight estimation system according to any one of claims 1 to 5, wherein

   the imaging unit images a plurality of tunas whose body weight is to be measured in a batch as a subject by a suspension scale,
   the individual body length measurement unit measures a fork length, a total length, or a body length of each tuna as the first length from the captured image,
   the body weight rate calculation unit calculates the body weight rate of each tuna from the measured first length of each tuna, and
   the body weight estimation unit estimates the body weight of each tuna based on the body weight of the plurality of tunas which is measured in a batch and the body weight rate of each tuna.

7. An individual body weight estimation method comprising:

   imaging a plurality of individuals whose body weight is to be measured in a batch as a subject,
   measuring a first length indicating a size of each individual included in the plurality of individuals from an captured image,
   calculating a body weight rate of each individual from the measured first length of each individual, and
   estimating a body weight of each individual, based on the body weight of the plurality of individuals and the body weight rate of each individual.

8. The individual body weight estimation method according to claim 7, wherein
   the body weight of each individual is estimated from the measured first length of each individual, and the weight rate of each individual is calculated from the estimated body weight of each individual.

9. An individual body weight estimation program causing a computer to execute:

an individual length measurement process of measuring a first length indicating a size of each individual included in a plurality of individuals from an image, captured by an imaging unit, with the plurality of individuals whose body weight is to be measured in a batch as a subject,

a body weight rate calculation process of calculating a body weight rate of each individual from the measured first length of each individual, and

a body weight estimation process of estimating a body weight of each individual based on the body weight of the plurality of individuals and the body weight rate of each individual.

10. The individual body weight estimation program according to claim 9, causing the computer to execute estimating the body weight of each individual from the measured first length of each individual, and calculating the weight rate of each individual from the estimated body weight of each individual, in the body weight rate calculation process.

# FIG. 1

100

INDIVIDUAL BODY WEIGHT ESTIMATION SYSTEM

10

BODY WEIGHT MEASUREMENT UNIT

20

IMAGING UNIT

30

INDIVIDUAL BODY LENGTH
MEASUREMENT UNIT

40

BODY WEIGHT RATIO
CALCULATION UNIT

50

BODY WEIGHT ESTIMATION UNIT

# FIG. 2

```
            ┌─────────────────────┐
            │        START         │
            └─────────────────────┘
                      │
                      ▼
┌─────────────────────────────────────────────────────────┐   S11
│   MEASURE BODY WEIGHT OF PLURALITY OF INDIVIDUALS         │
│            IN A BATCH BY HANGING                          │
└─────────────────────────────────────────────────────────┘
                      │
                      ▼
┌─────────────────────────────────────────────────────────┐   S12
│   IMAGE PLURALITY OF MEASURED INDIVIDUALS AS SUBJECT      │
└─────────────────────────────────────────────────────────┘
                      │
                      ▼
┌─────────────────────────────────────────────────────────┐   S13
│  MEASURE FIRST LENGTH INDICATING SIZE OF EACH INDIVIDUAL  │
│ INCLUDED IN PLURALITY OF INDIVIDUALS FROM CAPTURED IMAGE  │
└─────────────────────────────────────────────────────────┘
                      │
                      ▼
┌─────────────────────────────────────────────────────────┐   S14
│     CALCULATE BODY WEIGHT RATE OF EACH INDIVIDUAL         │
│    FROM MEASURED FIRST LENGTH OF EACH INDIVIDUAL          │
└─────────────────────────────────────────────────────────┘
                      │
                      ▼
┌─────────────────────────────────────────────────────────┐   S15
│        ESTIMATE BODY WEIGHT OF EACH INDIVIDUAL            │
│   BASED ON BODY WEIGHT OF PLURALITY OF INDIVIDUALS        │
│      AND BODY WEIGHT RATE OF EACH INDIVIDUAL              │
└─────────────────────────────────────────────────────────┘
                      │
                      ▼
            ┌─────────────────────┐
            │         END          │
            └─────────────────────┘
```

# FIG. 3

INDIVIDUAL BODY WEIGHT ESTIMATION SYSTEM — 200

- BODY WEIGHT MEASUREMENT UNIT — 10
- IMAGING UNIT — 20
- INDIVIDUAL BODY LENGTH MEASUREMENT UNIT — 30
- BODY WEIGHT RATIO CALCULATION UNIT — 40
- WARNING OUTPUT UNIT — 41
- BODY WEIGHT ESTIMATION UNIT — 50

# FIG. 4

START

↓

MEASURE BODY WEIGHT OF PLURALITY OF INDIVIDUALS IN A BATCH BY HANGING ⟩ S11

↓

IMAGE PLURALITY OF MEASURED INDIVIDUALS AS SUBJECT ⟩ S12

↓

MEASURE FIRST LENGTH INDICATING SIZE OF EACH INDIVIDUAL INCLUDED IN PLURALITY OF INDIVIDUALS FROM CAPTURED IMAGE ⟩ S13

↓

ESTIMATE BODY WEIGHT OF EACH INDIVIDUAL FROM MEASURED FIRST LENGTH OF EACH INDIVIDUAL ⟩ S21

↓

CALCULATE BODY WEIGHT RATE OF EACH INDIVIDUAL FROM ESTIMATED BODY WEIGHT OF EACH INDIVIDUAL ⟩ S22

↓

DIFFERENCE BETWEEN SUM OF ESTIMATED BODY WEIGHTS AND BODY WEIGHT OF PLURALITY OF MEASURED INDIVIDUALS EXCEEDS PREDETERMINED THRESHOLD VALUE ? ⟩ S23

YES → OUTPUT WARNING ⟩ S24

NO

↓

ESTIMATE BODY WEIGHT OF EACH INDIVIDUAL BASED ON BODY WEIGHT OF PLURALITY OF INDIVIDUALS AND BODY WEIGHT RATE OF EACH INDIVIDUAL ⟩ S15

↓

END

# FIG. 5

80

INDIVIDUAL BODY WEIGHT ESTIMATION SYSTEM

81

IMAGING UNIT

82

INDIVIDUAL BODY LENGTH
MEASUREMENT UNIT

83

BODY WEIGHT RATIO
CALCULATION UNIT

84

BODY WEIGHT ESTIMATION UNIT

**EP 4 036 536 A1**

<table>
<tr><td colspan="2" align="center"><strong>INTERNATIONAL SEARCH REPORT</strong></td><td colspan="2">International application No.<br>PCT/JP2020/024583</td></tr>
</table>

**A.  CLASSIFICATION OF SUBJECT MATTER**
G01G 17/00(2006.01)i; G01G 9/00(2006.01)i; G01G 23/35(2006.01)i
FI: G01G17/00 C; G01G9/00; G01G23/35

According to International Patent Classification (IPC) or to both national classification and IPC

**B.  FIELDS SEARCHED**

Minimum documentation searched (classification system followed by classification symbols)
G01G1/00-23/48; G01B11/00-11/30

| Documentation searched other than minimum documentation to the extent that such documents are included in the fields searched | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| A | JP 2016-176795 A (DAINIPPON PRINTING CO., LTD.) 06.10.2016 (2016-10-06) entire text, all drawings | 1-10 |
| A | CN 105318944 A (ZHEJIANG UNIVERSITY) 10.02.2016 (2016-02-10) entire text, all drawings | 1-10 |
| A | JP 2011-53070 A (KAWANISHI, Katsuzo) 17.03.2011 (2011-03-17) entire text, all drawings | 1-10 |
| A | JP 2002-5637 A (SUMITOMO METAL MINING CO., LTD.) 09.01.2002 (2002-01-09) entire text, all drawings | 1-10 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 July 2020 (09.07.2020) | 21 July 2020 (21.07.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**14**

| INTERNATIONAL SEARCH REPORT Information on patent family members | | International application No. PCT/JP2020/024583 | |
|---|---|---|---|
| Patent Documents referred in the Report | Publication Date | Patent Family | Publication Date |
| JP 2016-176795 A | 06 Oct. 2016 | (Family: none) | |
| CN 105318944 A | 10 Feb. 2016 | (Family: none) | |
| JP 2011-53070 A | 17 Mar. 2011 | (Family: none) | |
| JP 2002-5637 A | 09 Jan. 2002 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019045089 A

- JP 2019172491 A